Europäisches Patentamt

**European Patent Office**

**Office européen des brevets**

(19)

(11) Numéro de publication: **0 202 696**

**B1**

(12) **FASCICULE DE BREVET EUROPÉEN**

(45) Date de publication du fascicule du brevet:
**12.07.89**

(21) Numéro de dépôt: **86200678.0**

(22) Date de dépôt: **22.04.86**

(51) Int. Cl.⁴: **A 61 M 5/14**

(54) **Dispositif implantable à action manuelle pour la distribution séquentielle de doses d'une substance, notamment thérapeutique.**

(30) Priorité: **21.05.85 FR 8508068**

(43) Date de publication de la demande:
**26.11.86 Bulletin 86/48**

(45) Mention de la délivrance du brevet:
**12.07.89 Bulletin 89/28**

(84) Etats contractants désignés:
**DE FR GB IT NL SE**

(56) Documents cité:
**EP-A-0 143 503**
**FR-A-2 458 287**
**US-A-3 595 232**
**US-A-4 013 074**
**US-A-4 258 711**

(73) Titulaire: **APPLIED PRECISION LIMITED, 100 Fetter Lane, London EC4A 1DD (GB)**

(72) Inventeur: **Lazorthes, Guy, 26 rue d'Auriol, F-31400 Toulouse (FR)**

(74) Mandataire: **Barre, Philippe, Cabinet Barre- Gatti-Laforgue 95 rue des Amidonniers, F-31069 Toulouse Cédex (FR)**

EP 0 202 696 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

L'invention concerne un dispositif de distribution séquentielle destiné à être totalement implanté dans une région sous-cutanée accessible du corps d'un patient en vue de permettre de distribuer des doses d'une substance liquide (essentiellement thérapeutique); elle vise plus particulièrement un dispositif du type comprenant un réservoir de stockage de plusieurs doses, un site de remplissage par ponction percutanée en vue de recharger ledit réservoir, et une pompe d'injection permettant de refouler une dose dans un cathéter qui aboutit au compartiment corporel dans lequel la substance est à injecter, tel que défini dans le préambule de la revendication 1 et connu de US-A-4 013 074.

De nombreux dispositifs implantables de ce type ont été récemment conçus; au cours d'une intervention chirurgicale, ces dispositifs sont disposés sous le tissu sous-cutané de façon que leur site de remplissage soit accessible à travers la peau, le cathéter étant implanté entre le dispositif et le compartiment corporel concerné. Il est ainsi possible de délivrer sélectivement une dose précise de substance (encore désignée par "embole") au niveau de la cible visée, en atteignant des concentrations pharmacologiques seulement à ce niveau et des concentrations négligeables ailleurs; le stockage d'une pluralité d'emboles rend le dispositif autonome pour plusieurs injections.

Dans certains dispositifs, les pompes sont actionnées par des moyens automatisés permettant de programmer les injections (pompes électromagnétiques: brevets FR-2 537 440, WO 81/00888,...: pompes à moteur électrique: brevets EP-112 585, US-4 447 224, FR-2 354 105, EP 25005, WO 80/01755, FR-2 195 461, US-3 692 027...; pompes thermodynamiques ou à énergie chimique: brevets FR-2 528 313, CA-946 696, FR-2 370 481, US-3 731 681...; pompes osmotiques: ou électro-osmotiques: brevets FR-2 390 177, FR-2 354 106, US-4 340 054, US-4 300 557, US-3 995 631...). Toutefois, il s'agit là de dispositifs complexes, très onéreux, volumineux et souvent non totalement implantables.

On connaît également des dispositifs dans lesquels la pompe est actionnée manuellement par le malade lui-même ou le praticien afin d'assurer l'injection d'une embole. Par exemple, le brevet US-4 013 074 décrit un dispositif de ce type comprenant, dans une enceinte rigide, d'une part un réservoir de liquide et un réservoir de gaz séparés par un diaphragme, d'autre part une pompe péristaltique qui peut être actionnée manuellement à travers la peau grâce à un système mécanique approprié. Les dispositifs à action manuelle de ce type sont généralement plus simples que les dispositifs automatisés évoqués précédemment; de plus, ils permettent d'administrer le médicament à la demande de façon beaucoup plus souple, ce qui est intéressant dans de nombreux cas (notamment pour l'injection d'opioïdes anti-douleur). Toutefois, les dispositifs existants de ce type demeurent relativement complexes et donc onéreux (par exemple en raison de la présence d'organes mécaniques mobiles pour le dispositif ci-dessus cité), engendrent généralement une gêne pour le patient lorsqu'ils sont implantés dans les tissus et sont parfois difficiles à implanter ou à mettre en oeuvre (certains exigeant l'exécution de plusieurs opérations dans un ordre déterminé pour assurer l'injection d'une dose).

La présente invention vise un nouveau dispositif de distribution séquentielle du type à action manuelle, destiné à être implanté dans une région sous-cutanée accessible en vue de la distribution séquentielle de doses ou emboles d'une substance liquide. L'invention vise essentiellement à fournir un dispositif de structure très simplifiée, bénéficiant d'un coût réduit par rapport à tous les dispositifs de ce type connus actuellement.

Un autre objectif essentiel de l'invention est de fournir un dispositif ayant un réservoir capable de contenir un nombre de doses accru en vue d'augmenter son autonomie, tout en présentant un faible encombrement et une forme propre à faciliter grandement son implantation.

Un autre objectif est de fournir un dispositif apte à s'intégrer aux tissus sous-cutanés en épousant les formes locales de ceux-ci afin de réduire la gêne subie par le patient.

Un autre objectif est de fournir un dispositif de mise en oeuvre facile et sûre, apte à distribuer lors de chaque action sur la pompe une dose précise prédéterminée de substance.

Un autre objectif est de doter le dispositif d'un site accessoire d'accès pour l'injection chronique de doses de substances différentes de celle contenue dans le réservoir (administration complémentaire d'un autre médicament, vérification d'étanchéité par injection d'un produit opaque...).

Un autre objectif est de fournir un dispositif ne conduisant à aucun risque de perturbations ou d'artefacts lors d'explorations réalisées par des techniques d'imagerie moderne C tomodensitométrie, résonance magnétique...).

A cet effet, le dispositif visé par l'invention est du type comprenant:

- un réservoir de capacité correspondant à plusieurs doses,
- un site de remplissage du réservoir, comportant une chambre de remplissage, un septum d'injection de substance liquide dans ladite chambre et des moyens de communication entre la chambre de remplissage et le réservoir,
- et une pompe manuelle comportant une chambre volumétrique de capacité correspondant à une dose, des moyens de communication entre le réservoir et ladite chambre en vue du remplissage de cette dernière et des moyens de communication entre ladite chambre volumétrique et un cathéter extérieur.

Le dispositif conforme à la présente invention se caractérise en ce que:

(a) le réservoir est constitué par une poche souple composée d'une première paroi souple, dite externe, et d'une seconde paroi souple dite interne, s'étendant en regard l'une de l'autre et liées l'une à l'autre de façon étanche le long de leur bordure périphérique,

(b) la pompe manuelle comprend un corps rigide monobloc, lequel:

- est creusé dans son épaisseur d'une dépression de forme concave débouchant sur une face dudit corps, dite face active, en vue de former la chambre volumétrique,

- est interposé entre les deux parois souples de la poche formant le réservoir,

- porte une membrane de refoulement sur sa face active, fermant la chambre volumétrique et située du côté de la paroi souple externe,

- et est fixé par sa face opposée sur la paroi souple interne,

(c) les moyens de communication entre la chambre volumétrique et le cathéter sont équipés d'une valve unidirectionnelle à seuil de pression, adaptée pour ouvrir la communication de la chambre vers le cathéter lorsque ladite chambre est soumise à une surpression supérieure à un seuil Pc déterminé,

(d) les moyens de communication entre le réservoir et la chambre volumétrique sont équipés:

- d'une valve anti-retour interdisant le retour du liquide de la chambre vers le réservoir,

- d'une soupape de sécurité adaptée pour autoriser l'écoulement par gravité du réservoir vers la chambre volumétrique et pour obturer la communication si la surpression dans le réservoir dépasse une valeur Pr inférieure ou égale au seuil Pc.

De préférence, la poche souple formant le réservoir est rigidifiée en bordure de ses parois souples par un cadre périphérique fixé sur le pourtour desdites parois souples.

Selon une autre caractéristique de l'invention, le site de remplissage du réservoir est interposé entre les deux parois souples, à proximité de la bordure périphérique de celles-ci dans une zone de la poche opposée à celle où est située la pompe manuelle; ce site comprend un anneau portant le septum d'injection, lequel est de préférence constitué par deux membranes élastiques séparées par un vide d'air (cette disposition réalise une sorte de sas qui améliore l'étanchéité du site de remplissage après un grand nombre de perforations). Il est également possible de prévoir un septum d'injection réalisé par la technique connue "Plug Silicone".

Ainsi, le dispositif de l'invention est totalement intégré dans une poche souple qui en définit la forme et la contexture externe. Il est totalement dépourvu de tout organe mécanique, l'injection s'effectuant directement par un refoulement provoqué par la membrane sous l'effet d'une pression manuelle exercée sur celle-ci à travers la peau du patient. Un tel dispositif présente une structure très simple, et sa fabrication (en particulier par empilage et collage des divers éléments constitutifs, comme on le verra plus loin) est d'un coût très réduit par rapport aux dispositifs antérieurs. De plus, la poche souple est facile à insérer dans les tissus sous-cutanés lors de l'implantation. Sa souplesse la rend apte à épouser la forme locale des tissus avec une gêne très limitée pour le patient (contrairement aux dispositifs antérieurs à enceinte rigide). Le cadre périphérique maintient la poche dans le sens radial mais laisse au dispositif la liberté de se déformer perpendiculairement à son plan pour s'intégrer parfaitement aux tissus contigus. En outre, la capacité du réservoir formé par la poche souple peut correspondre à un nombre de doses relativement important (de l'ordre de 80 par exemple, en raison, d'une part, de la conformation même dudit réservoir, d'autre part, de la place négligeable occupée par les autres organes (pompe et site de remplissage). Par ailleurs, l'injection d'une embole s'effectue par une manoeuvre unique que le malade peut exécuter lui-même, consistant à repousser la membrane de refoulement dans la dépression du corps, jusqu'au fond de celle-ci; cette dépression présente de préférence une forme correspondant à l'empreinte d'un index. Cette manoeuvre facile à exécuter provoque l'injection d'une dose précise de substance, correspondant au volume de la chambre volumétrique délimitée par la dépression. Le fonctionnement du dispositif conforme à l'invention est très sûr: en particulier, comme on le comprendra mieux plus loin, aucune injection involontaire ne peut se produire, par exemple en cas de pression accidentelle exercée sur la poche souple formant le réservoir.

Par ailleurs, selon un mode de réalisation préférentiel, la poche souple formant le réservoir présente une forme générale triangulaire; la pompe manuelle est située à proximité d'un des sommets de celle-ci et le site de remplissage à proximité d'un autre sommet. Lors de l'implantation, la poche peut être facilement positionnée de sorte que la pompe se trouve en partie basse et le site de remplissage en partie haute; ces dispositions permettent un auto-remplissage par gravité de la chambre volumétrique de celle-ci, et en cas de présence d'air dans le réservoir, elles assurent la remontée de cet air vers le site de remplissage à partir duquel ledit air peut être purgé.

Selon une autre caractéristique de l'invention, la membrane élastique de refoulement est sensiblement plane au repos et est collée de façon étanche autour de la dépression du corps sur une bordure prévue à cet effet. Cette disposition (qui va dans le sens d'une réduction du coût de fabrication) donne à la membrane une aptitude naturelle à se déformer pour épouser le fond de la dépression du corps sous l'action d'une pression manuelle.

Les divers éléments constitutifs du dispositif de l'invention (parois souples, cadre de rigidification, septum d'injection, corps de pompe, membrane de refoulement...) sont réalisés en des matériaux biocompatibles dont des exemples seront donnés plus loin; l'ensemble est, de préférence, entièrement recouvert d'un mince revêtement souple en une matière biocompatibles, en particulier en silicone, qui accroît encore l'étanchéité et la biocompatibilité du dispositif.

En outre, la membrane de refoulement de la pompe est avantageusement réalisée en une matière élastique du type "auto-étanche" ("Self-Sealing") en vue de conférer à la pompe une fonction accessoire de site d'accès d'injection chronique. Le médicament principal contenu dans le réservoir peut ainsi être injecté sur demande, mais le praticien garde la faculté d'injecter en cas de besoin une autre substance par simple ponction percutanée utilisant le dispositif, sans avoir à réaliser une injection plus délicate vers le compartiment corporel concerné (telle qu'injection intraveineuse, intra-artérielle, intra-rachidienne, intra-ventriculaire cérébrale, intra-péritonale...). Ce site d'accès accessoire peut également être utilisé pour l'injection de produits opaques en vue de vérifier l'étanchéité de la pompe et du cathéter.

D'autres caractéristiques, buts et avantages de l'invention ressortiront de la description qui suit en référence aux dessins annexés, qui en présentent un mode de réalisation préférentiel; sur ces dessins qui font partie intégrante de la présente description:

- la figure 1 est une vue en pespective à échelle dilatée d'un dispositif conforme à l'invention,
- la figure 2 en est une coupe par un plan AA' parallèle à son côté long (h),
- la figure 3 en est une coupe par un plan BB' parallèle à son côté court (v),
- la figure 4 en est une coupe de détail, à échelle plus grande, par un plan C (parallèle au plan AA'),
- la figure 5 en est une coupe de détail (à même échelle que la figure 4) par un plan D parallèle au plan BB',
- la figure 6a schématise l'implantation du dispositif en position latérale droite dans la paroi abdominale d'un patient, cependant que les figures 6b et 6c en illustrent l'utilisation,
- la figure 7 schématise l'implantation d'un dispositif symétrique, en position latérale gauche dans la paroi abdominale d'un patient.

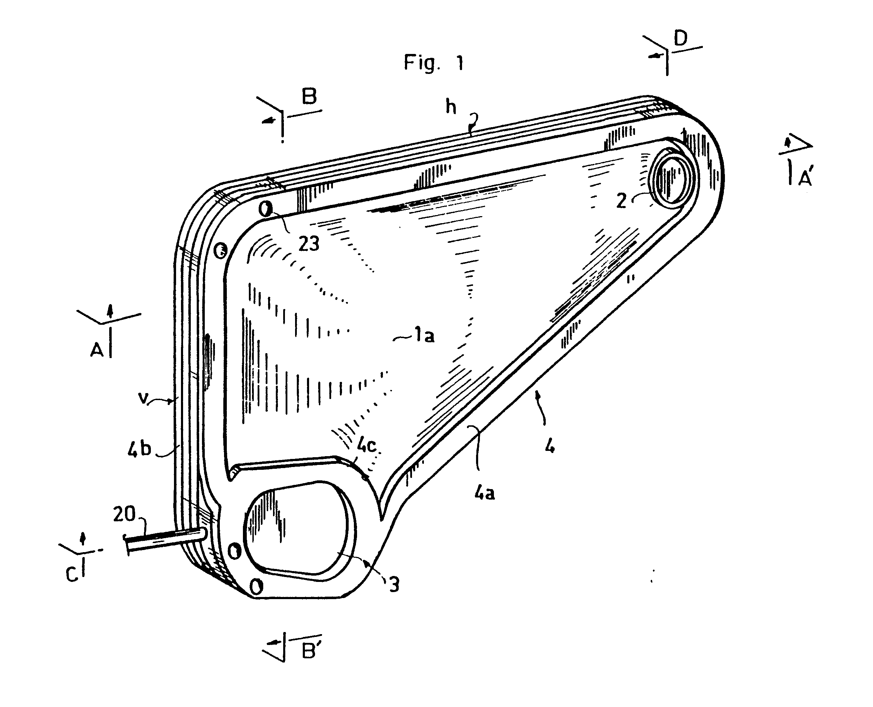

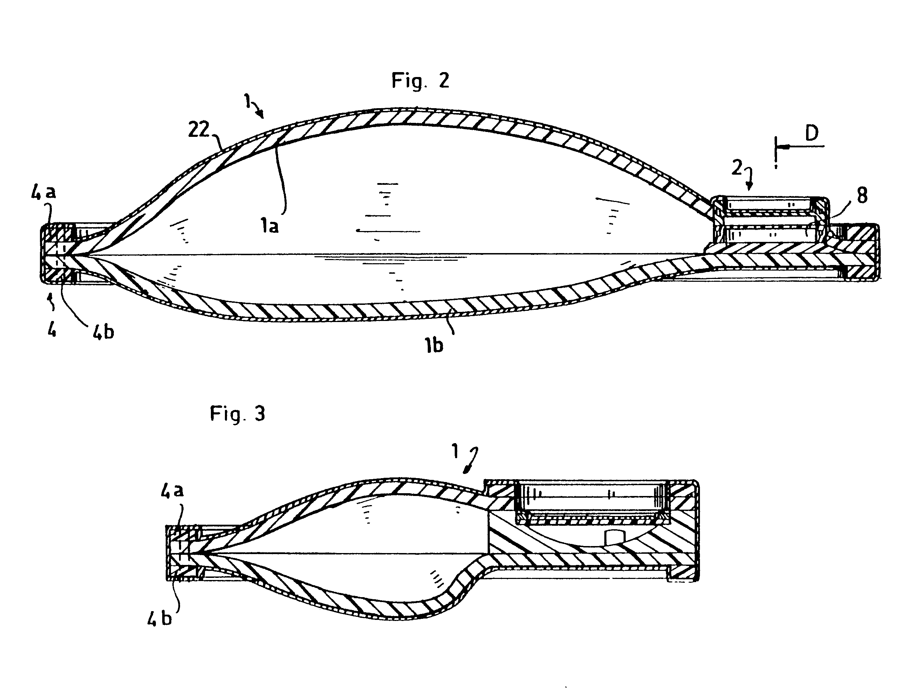

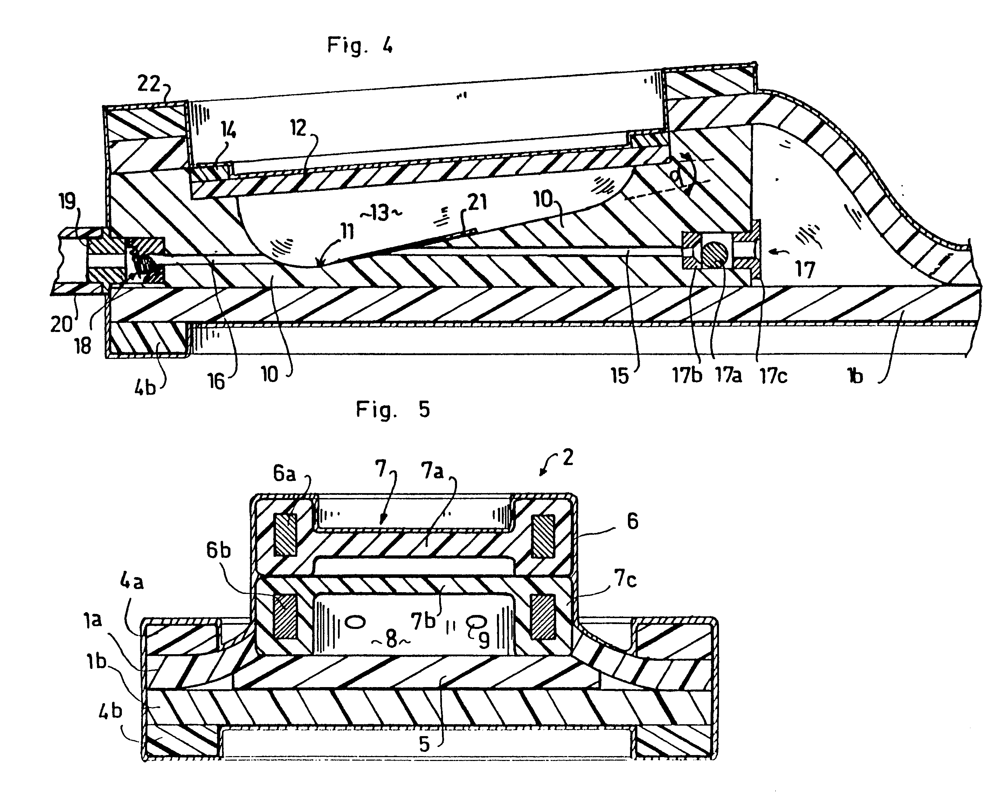

Le dispositif représenté aux figures 1 à 5 (à échelle de l'ordre de 1,75 pour les figures 2 et 3 et à échelle de l'ordre de 4 pour les figures 4 et 5) est destiné à être implanté dans les tissus sous-cutanés en vue d'autoriser une injection séquentielle, par unité, d'une dose de substance thérapeutique (embole) dans un compartiment corporel, par exemple intrarachidien, tout en étant rechargeable par une simple ponction percutanée au terme de sa période d'autonomie.

Ce dispositif comprend une poche souple 1 composée de deux parois souples 1a et 1b, l'une (1a) dite externe, destinée à être orientée du côté de la peau lors de l'implantation, l'autre (1b) dite interne, tournée vers les tissus internes. Chaque paroi 1a ou 1b est préformée en matière synthétique souple biocompatible, notamment en silicone renforcé de "Dacron", de façon à présenter au repos la section d'un dôme, comme le montrent les figures. La paroi externe 1a présente une courbure plus accentuée que la paroi interne 1b qui est sensiblement plus plate, afin d'améliorer l'intégration du dispositif dans les tissus.

En plan, ces parois souples présentent une forme générale triangulaire avec des sommets arrondis. En particulier, elles sont agencées de sorte que la poche 1 possède la forme générale d'un triangle approximativement rectangle, avec un côté -v- de l'angle droit plus court que l'autre côté -h-.

Ces parois souples 1a et 1b sont étanchément collées sur leur périphérie l'une contre l'autre, avec interposition, d'une part, d'un site de remplissage 2 dans l'angle aigu délimité par le côté long -h- et l'hypothénuse, à proximité du sommet correspondant, d'autre part, d'une pompe manuelle 3 dans l'angle aigu délimité par le côté court -v- et l'hypothénuse, à proximité du sommet correspondant.

Il est à noter que les deux parois de la poche 1 pourraient être solidarisées au moment de leur moulage de façon à réaliser directement une poche d'un seul tenant.

La poche souple 1 ainsi formée est rigidifiée en bordure de ses parois souples par un cadre périphérique semi-rigide 4, composé de deux éléments 4a et 4b fixés par collage en regard l'un de l'autre respectivement sur la paroi externe 1a et sur la paroi interne 1b. Le cadre est arrondi au niveau des angles pour épouser la forme des parois souples et est réalisé en polytétrafluoroéthylène, de façon à maintenir la poche souple dans le sens radial, tout en lui laissant une liberté de déformation selon une direction perpendiculaire à son plan.

L'élément 4a du cadre qui est fixé sur la paroi souple externe 1a possède une extension 4c s'étendant autour de la pompe 3.

La poche souple 1, rigidifiée par son cadre périphérique 4, possède, à l'état de plénitude, une forme biconvexe dissymétrique, d'épaisseur faible par rapport à ses autres dimensions, qui se prête à une insertion aisée dans les tissus sous-cutanés et est apte à épouser la forme des tissus. La capacité du réservoir qu'elle forme, peut atteindre entre 30 cm³ et 50 cm³ sans aucun risque de gêne pour le patient. Une embole d'opioïde correspond généralement entre 0,4 et 0,6 cm³, de sorte que l'autonomie moyenne du dispositif peut aisément atteindre 80 à 100 emboles.

Par ailleurs, le site de remplissage 2 qui est représenté en détail à la figure 5 est composé d'une plaque rigide de butée 5 fixée par collage sur la paroi souple linterne 1b et d'un anneau 6 solidaire de ladite plaque et portant un septum d'injection 7. La paroi souple externe 1a comporte une fenêtre au niveau de l'anneau de sorte que le septum 7 vienne en correspondance avec cette fenêtre et soit directement accessible; la paroi externe 1a est collée étanchement autour de l'anneau 6.

L'anneau 6, son septum 7 et la plaque de butée 5 délimitent une chambre de remplissage 8 qui communique avec le volume intérieur de la poche souple 1 par deux trous tels que 9 percés dans l'anneau 6.

En l'exemple, le septum 7 est constitué par deux membranes élastiques 7a et 7b, séparées par un vide d'air et solidaires chacune d'un bourrelet périphérique tels que 7c, cependant que l'anneau 6 comprend deux éléments annulaires 6a et 6b qui forment l'âme des bourrelets périphériques de membrane 7c.

La plaque de butée 5 est réalisée en un matériau rigide biocompatible, notamment polycarbonate; les membranes 7a et 7b et leurs bourrelets 7c sont réalisés en un matériau élastique biocompatible, notamment en silicone auto-étanche ("self sealing") les éléments 6a et 6b de l'anneau sont réalisés en un matériau rigide biocompatible, notamment polytétrafluoroéthylène.

Les membranes 7a et 7b et leurs bourrelets 7c sont moulés avec insertion des éléments 6a et 6b de l'anneau, et l'ensemble est empilé et étanchement collé sur la plaque de butée 5.

Le remplissage du réservoir formé par la poche 1 est assuré au moyen d'une aiguille d'injection piquée à travers le septum 7 jusqu'au contact de la plaque 5; le liquide injecté dans la chambre 8 s'écoule par les trous de communication 9 dans la poche souple 1 jusqu'à remplissage de celle-ci. Les deux membranes séparées 7a et 7b qui forment le septum constituent un sas fournissant une excellente étanchéité après de nombreuses perforations.

Bien entendu, le septum d'injection 7 peut recevoir d'autres formes de réalisation et, en particulier, être réalisé par la technique de moulage connue sous le terme "Plug Silicone".

Par ailleurs, la pompe manuelle 3 située dans l'autre angle aigu de la poche 1 est appelée à se positionner après implantation en partie basse de ladite poche, de façon que le liquide ait tendance à s'écouler par gravité vers cette pompe. Le remplissage de ladite pompe s'effectuera donc, d'une part, par gravité, d'autre part, par aspiration après la manoeuvre d'injection vidant celle-ci.

Cette pompe 3, qui est représentée en détail à la figure 3, comprend un corps rigide 10 moulé d'un seul tenant en matière synthétique rigide biocompatible, notamment polycarbonate ou silicone renforcé de "Dacron" ou polyéthérisulfome; ce corps présente une épaisseur de l'ordre de 10 mm plus faible que ses autres dimensions; vu en plan, il possède une forme oblongue symétrique par rapport au plan axial (C) telle que représentée à la figure 1, avec un chant périphérique arrondi sur tout son pourtour.

La face supérieure du corps, dite face active, est légèrement inclinée par rapport à la face opposée de sorte que le corps présente une épaisseur décroissante lorsque l'on s'éloigne du réservoir 1.

Sur sa face active, le corps 1 est creusé d'une dépression 11, de profondeur faible par rapport aux dimensions de sa section d'ouverture sur la face active.

Cette dépression 11 possède une profondeur progressivement croissante vers sa portion 1a plus profonde située du côté opposé au réservoir 1; elle présente une forme correspondant à l'empreinte d'un index: forme oblongue, symétrique par rapport au plan C, dont les sections par des plans de coupe parallèles à la face active possèdent une surface décroissante au fur et à mesure que ces plans de coupe se rapprochent du fond de la dépression.

En l'exemple, la portion centrale du fond de la dépression 11 est de surface conique et se prolonge de chaque côté par des surfaces arrondies jusqu'à la section d'ouverture sur la face active; ce fond est incliné parallèlement au plan C vers la partie la plus profonde de façon à former, dans le plan C, un angle $\alpha$ avec la face active sensiblement compris entre 10° et 15°.

La face du corps opposée à la face active est collée sur la paroi souple interne 1b, cependant que la face active porte une membrane de refoulement 12 qui ferme la dépression 11 en vue de délimiter une chambre volumétrique 13.

La membrane de refoulement 12 est sensiblement plane au repos et est inclinée comme la face active du corps vers le côté où est appelé à être raccordé le cathéter 20; cette membrane est fixée par collage, de façon étanche, autour de la dépression 11 sur une bordure périphérique que comporte le corps. La membrane 12 est découpée à la forme de ladite bordure périphérique dans une matière synthétique biocompatible, notamment silicone de préférence "auto-étanche" ("Self-Sealing") de façon à pouvoir être traversée par une aiguille afin de constituer un site accessoire d'injection. En l'exemple, une bague de maintien 14 en polytétrafluoroéthylène est collée au-dessus et en bordure de la membrane 12. Il est à noter que, comme pour le site de remplissage 2, la membrane 12 peut être réalisée par la technique de "Plug Silicone".

La paroi souple 1a est pourvue d'une fenêtre en correspondance avec la membrane 12 de façon à rendre celle-ci directement accessible; cette paroi souple 1a est collée, en bordure de sa fenêtre sur le corps 10. L'extension 4c du cadre périphérique qui est elle-même collée, en superposition, sur la paroi souple 1a, forme un élément plus dur, qui permet au toucher de repérer facilement la membrane 12 afin de la repousser avec l'index à travers la peau du patient; cette action permet de déformer la membrane 12 jusqu'à l'amener à épouser le fond de la dépression 11 sur toute sa surface, afin de refouler la

totalité du liquide contenu dans la chambre volumétrique 13.

La dépression 11 possède en l'exemple un volume sensiblement compris entre 0,4 et 0,6 $cm^3$, notamment égal à 0,5 $cm^3$, en vue de délimiter avec la membrane de refoulement au repos une chambre volumétrique de capacité correspondant à une embole.

En outre, le corps de pompe 10 est percé, dans son épaisseur, de deux canaux opposés 15 et 16 qui s'étendent parallèlement aux faces du corps et débouchent, d'une part, dans la dépression 11 au voisinage du fond de celle-ci, d'autre part, sur le chant périphérique du corps à l'extérieur de celui-ci.

L'un des canaux 15 est situé du côté de la poche souple de façon à déboucher entre les parois souples 1a et 1b à l'intérieur de ladite poche, dans la partie la plus basse de celle-ci. L'autre canal 16, diamétralement opposé au premier, débouche à l'extérieur de la poche sur la partie extérieure du chant périphérique du corps 10.

Chacun de ces canaux possède un tronçon de diamètre plus important, débouchant sur le pourtour du corps (soit dans la poche pour le canal 15, soit à l'extérieur de celle-ci pour le canal 16).

Dans le tronçon du canal 15, est logée une soupape de sécurité 17, adaptée pour obturer le canal si la surpression dans la poche souple 1 dépasse une valeur Pr déterminée, en particulier comprise entre 20 et 30 millibars.

Cette soupape de type connu en soi peut être composée d'une bille mobile 17a en matériau biocompatible, notamment en polytétrafluoroéthylène, d'un siège de soupape 17b réalisé en matériau biocompatible, notamment silicone, et d'une butée arrière 17c réalisée en matériau biocompatible, notamment polytétrafluoroéthylène.

La bille possède une faible course axiale (de l'ordre de 1 mm) et son poids est adapté pour autoriser un écoulement du liquide de la poche 1 vers la chambre volumétrique 13, tant que la différence de pression entre ladite poche et ladite chambre demeure inférieure à la valeur Pr et pour se déplacer vers le siège et obturer celui-ci lorsque cette valeur est atteinte ou dépassée. En raison de la faible course axiale de la bille, le volume de liquide qui traverse le canal 15 en cours de fermeture lorsque la limite de pression est atteinte, est très faible (de l'ordre de 0,2 $mm^3$), ce qui entraîne une augmentation négligeable du volume de la chambre volumétrique 13 (par déformation vers le haut de la membrane de refoulement 12).

Bien entendu, la soupape de sécurité 17 peut revêtir toute autre forme connue en elle-même.

Par ailleurs, dans le tronçon de diamètre élargi du canal opposé 16, est logée une valve unidirectionnelle à seuil 18, adaptée pour ouvrir le canal si la chambre 13 est soumise à une surpression supérieure à un seuil Pc et pour obturer ledit canal dans le cas contraire; ce seuil Pc est prévu supérieur ou égal à la valeur Pr précitée et est en particulier compris entre 50 et 60 millibars.

La valve 18 est disposée dans le canal 16, en butée contre un raccord 19 collé dans ledit canal et auquel est connecté par collage un cathéter 20.

Cette valve unidirectionnelle peut être une valve de type à bille et ressort (en matière synthétique biocompatible) telle que schématisée, ou de toute autre valve de type connu.

De plus, une valve anti-retour 21, constituée en l'exemple par une languette élastique collée au fond de la dépression de façon à pouvoir masquer l'orifice du canal 15, écarte toute possibilité de retour de liquide de la chambre volumétrique 13 vers le réservoir 1.

La chambre 13 se remplit de liquide à travers le canal 15, soit par gravité, soit par aspiration provoquée par le retour de la membrane 12 après une pression sur celle-ci. Une surpression accidentelle qui apparaîtrait dans le réservoir (par compression involontaire de la poche souple 1 qui forme celui-ci), ne peut en aucun cas engendrer une injection inopportune à travers le cathéter 20. En effet, si cette surpression demeure inférieure à la valeur Pr, la soupape de sécurité 17 reste ouverte mais la valve unidirectionnelle garde son état fermé (Pr < Pc); si cette surpression devient supérieure à la valeur Pr, il y a amorçage d'un écoulement de liquide dans le canal 15 (qui provoque une très légère augmentation du volume de la chambre 13) et la soupape de sécurité 17 se ferme par application de la bille 17a sur son siège 17b, évitant une augmentation sensible de la pression dans la chambre volumétrique (la bille restant en appui sur son siège tant que la surpression dans la poche demeure supérieure à Pr).

L'injection d'une dose est assurée par une pression exercée sur la membrane de refoulement 12 par l'opérateur jusqu'à amener la membrane en appui contre le fond de la dépression 11 délimitant la chambre volumétrique 13; une pression élevée s'établit alors dans ladite chambre 13 et provoque l'ouverture de la valve unidirectionnelle 18. La valve anti-retour 21 interdit tout refoulement vers le réservoir 1.

Par ailleurs, toute la surface externe du dispositif est avantageusement recouverte d'un mince revêtement souple et élastique 22, en particulier en silicone. Ce revêtement est réalisé après assemblage et collage des divers éléments constitutifs du dispositif, par tout procédé connu: projection, trempage... Il améliore la biocompatibilité du dispositif et accroît son étanchéité.

De plus, des trous de suture tels que 23 sont percés en bordure de la poche souple, à travers ladite poche, le cadre périphérique 4 et le revêtement 22. Ces trous sont positionnés, d'une part, à proximité de l'angle droit, d'autre part, à proximité de l'angle aigu inférieur, en bordure de la pompe manuelle 3. En l'exemple, ces trous sont prévus au nombre de quatre.

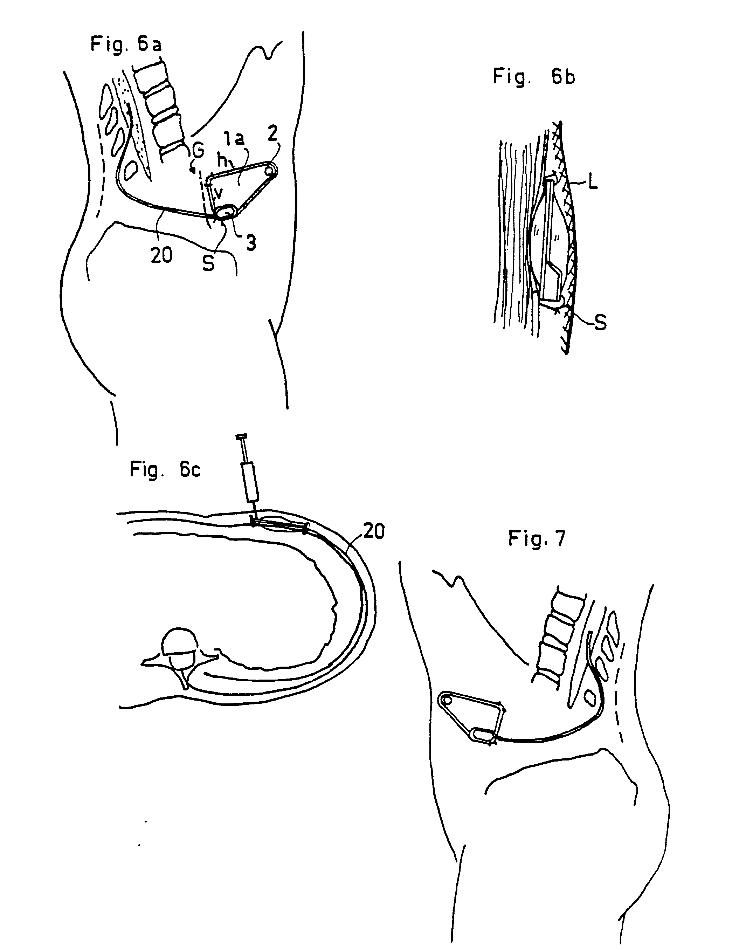

Le dispositif décrit est totalement implantable dans une zone sous-cutanée, comme l'illustrent les figures 6a, 6b et 6c. A titre d'exemple, cette implantation peut être réalisée en position latérale dans la paroi abdominale ou latéro-thoracique du patient.

Le dispositif visé aux figures 1 à 5 est plus particulièrement destiné à une implantation en position latérale droite. A cet effet, la poche souple triangulaire 1 est géométriquement agencée de sorte que son grand côté (h) s'étende vers la droite (le site d'implantation 2 étant situé à droite) lorsque le dispositif est placé dans un plan vertical avec son petit côté (v) vertical et sa pompe manuelle 3 en partie basse et lorsqu'il est observé de face, côté paroi externe 1a.

Le dispositif est implanté comme le montre la figure 6a, après incision suivant une ligne G. Sa forme permet de le glisser facilement entre la ligne cutanée L et les tissus internes musculo-aponévrotiques M dans une poche sous-cutanée pratiquée au cours de l'implantation. Des sutures S traversant les trous 23 l'immobilisent parfaitement; il est à noter que ces sutures sont situées du côté du cathéter et écartent les risques de torsion de celui-ci à sa sortie du dispositif. De plus, le dispositif est décalé par rapport à l'incision G et à la ligne de suture qui referme celle-ci, et peut être mise en oeuvre (remplissage, injection) sans être gênée par celle-ci.

La poche souple 1 formant le réservoir est remplie de liquide par injection percutanée à travers le septum du site de remplissage 2; cette injection réalisée au moyen d'une aiguille appropriée correspond à un volume de liquide qui en l'exemple est de l'ordre de 80 emboles, ce qui confère au dispositif une autonomie importante. Il est à noter que le remplissage du réservoir 1 peut s'effectuer quelle que soit la position du patient.

Le remplissage de la chambre volumétrique 13 de la pompe manuelle s'effectue par aspiration ou par gravité, le patient ayant une position telle que la pompe se trouve en partie basse (position debout, assise, couchée légèrement relevée...).

L'injection d'une embole s'effectue par simple pression sur la membrane de refoulement à travers la peau; l'opérateur réalise cette pression au moyen de son index jusqu'à ressentir le fond rigide du corps de pompe; la totalité de la dose est alors expulsée dans le cathéter, ce qui permet de pratiquer à la demande des injections de grande précision. Comme indiqué plus haut, aucun risque d'injection involontaire n'est à craindre si une pression comprime la poche souple 1 (par exemple en raison de la position du patient couché sur le ventre). Ainsi l'injection d'une embole s'opère de façon précise en toute sécurité par une action unique, facile à exécuter (le cas échéant par le malade lui-même).

Par ailleurs, la présence d'un site d'accès accessoire formé par la pompe 3 est un avantage considérable en pratique, puisqu'il permet l'injection de substances différentes de celle contenue dans le réservoir (polychimiothérapie...).

Il est à noter que l'agencement du dispositif assure une remontée automatique des bulles d'air éventuellement présentes dans le réservoir 1, vers le site de remplissage 2, cet air pouvant être facilement ponctionné à travers le septum 7.

La simplicité structurelle du dispositif et l'absence de pièces mécaniques (pièces de transmission ou autre) lui confèrent un prix de revient très abaissé par rapport aux dispositifs connus, et une légèreté participant à réduire la gêne subie par le patient. En outre, le dispositif ne comporte aucun élément métallique et le patient peut faire l'objet d'explorations par des techniques d'imagerie moderne, sans risque de perturbations ou d'artefacts.

Dans le cas où l'implantation est à effectuer en position latérale gauche, le praticien utilisera un dispositif analogue, mais symétrique par rapport au précédent, comme l'illustre la figure 7. La poche triangulaire est alors géométriquement agencée de sorte que son grand côté s'étende vers la gauche (le site d'implantation étant situé à gauche) lorsque le dispositif est placé dans un plan vertical avec son petit côté vertical et sa pompe manuelle en partie basse et lorsqu'il est observé de face, côté paroi externe.

**Revendications**

1. Dispositif de distribution séquentielle à action manuelle, destiné à être implanté dans une région sous-cutanée accessible en vue de la distribution séquentielle de doses d'une substance liquide, du type comprenant:
   - un réservoir (1) de capacité correspondant à plusieurs doses,
   - un site de remplissage (2) du réservoir, comportant une chambre de remplissage (8), un septum (7) d'injection de substance liquide dans ladite chambre et des moyens (9) de communication entre la chambre de remplissage et le réservoir,
   - et une pompe manuelle (3) comportant une chambre volumétrique (13) de capacité correspondant à une dose, des moyens (15) de communication entre le réservoir et ladite chambre en vue du remplissage de cette dernière et des moyens (16) de communication entre ladite chambre volumétrique et un cathéter extérieur (20),

   ledit dispositif de distribution étant caractérisé en ce que:

   (a) le réservoir est constitué par une poche souple (1) composée de deux parois, dite externe (1a), et dite interne (1b), s'étendant en regard l'une de l'autre et liées l'une à l'autre de façon étanche le long de leur bordure périphérique,
   (b) la pompe manuelle comprend un corps rigide monobloc (10), lequel:
      - est creusé dans son épaisseur d'une dépression (11) de forme concave débouchant sur une face dudit corps, dite face active, en vue de former la chambre volumétrique (13),

- est interposé entre les deux parois (1a, 1b) de la poche souple formant le réservoir,
- porte une membrane de refoulement (12) sur sa face active, fermant la chambre volumétrique et située du côté de la paroi externe (1a),
- et est fixé par sa face opposée sur la paroi interne,

(c) les moyens de communication entre la chambre volumétrique (13) et le cathéter (20) sont équipés d'une valve unidirectionnelle à seuil de pression (18) adaptée pour ouvrir la communication de la chambre vers le cathéter losque ladite chambre est soumise à une surpression supérieure à un seuil Pc déterminé,

(d) les moyens de communication entre le réservoir (1) et la chambre volumétrique (13) sont équipés:
- d'une valve anti-retour (21) interdisant le retour de liquide de la chambre vers le réservoir,
- d'une soupape de sécurité (17) adaptée pour autoriser l'écoulement du réservoir vers la chambre volumétrique et pour obturer la communication si la surpression dans le réservoir dépasse une valeur Pr inférieure ou égale au seuil Pc.

2. Dispositif de distribution selon la revendication 1, caractérisé en ce que:
- le corps de pompe (10) est interposé entre les deux parois souples (1a, 1b) à proximité de leur bordure périphérique,
- la paroi souple externe (1a) est pourvue en correspondance avec la membrane de refoulement d'une fenêtre et est fixée de façon étanche sur le corps (10) par les bords de ladite fenêtre.

3. Dispositif de distribution selon l'une des revendications 1 ou 2, caractérisé en ce que la poche souple (1) est rigidifiée en bordure de ses parois souples (1a, 1b) par un cadre périphérique (4) fixé sur le pourtour desdites parois souples.

4. Dispositif de distribution selon l'une des revendications 1, 2 ou 3, caractérisé en ce que:
- le site de remplissage (2) est disposé à proximité de la bordure périphérique des parois souples (1a, 1b) dans une zone de la poche souple opposée à celle où est située la pompe manuelle (3),
- ledit site de remplissage est composé d'une plaque rigide de butée (5) fixée sur la paroi souple interne (1b) et d'un anneau (6) solidaire de ladite plaque et portant le septum d'injection (7) de façon que celui-ci vienne en correspondance avec une fenêtre de la paroi souple externe (1a), laquelle est fixée de façon étanche autour dudit anneau.

5. Dispositif de distribution selon l'une des revendications 1, 2, 3 ou 4, caractérisé en ce que:
- la poche souple (1) formant le réservoir présente une forme générale triangulaire,
- la pompe manuelle (3) est située à proximité d'un des sommets et le site de remplissage (2) à proximité d'un autre sommet.

6. Dispositif de distribution selon les revendications 3 et 5 prises ensemble, caractérisé en ce que:
- chaque paroi souple (1a, 1b) est préformée en matière synthétique souple biocompatible, notamment silicone renforcé, de façon à présenter au repos la section d'un dôme,
- le cadre périphérique (4) comprend deux éléments (4a, 4b) en matière synthétique semi-rigide biocompatible, notamment polytétrafluoroéthylène, fixés en regard l'un de l'autre respectivement sur la paroi externe (1a) et sur la paroi interne (1b),
- le cadre (4) et les bordures des parois souples (1a, 1b) sont arrondis au niveau des angles.

7. Dispositif de distribution selon l'une des revendications 5 ou 6, caractérisé en ce que:
- la poche souple (1) formant le réservoir présente la forme générale d'un triangle approximativement rectangle avec un côté (v) de l'angle droit plus court que l'autre,
- la pompe manuelle (3) est située dans l'angle aigu délimité par le côté court (v) et l'hypothénuse, à proximité du sommet correspondant,
- le site de remplissage (2) est situé dans l'angle aigu délimité par le côté long (h) et l'hypothénuse, à proximité du sommet correspondant,
- des trous de suture (23) sont prévus en bordure de la poche souple, d'une part, à proximité de l'angle droit, d'autre part, à proximité de l'angle aigu en bordure de la pompe manuelle (3).

8. Dispositif de distribution selon la revendication 7, destiné à être implanté dans la paroi abdominale d'un patient en position latérale droite, caractérisé en ce que la poche triangulaire est géométriquement agencée de sorte que son grand côté (h) s'étende vers la droite (le site d'implantation (2) étant situé à droite) lorsque le dispositif est placé dans un plan vertical avec son petit côté (v) vertical et sa pompe menuelle (3) en partie basse et lorsqu'il est observé de face, côté paroi externe (1a).

9. Dispositif de distribution selon la revendication 7, destiné à être implanté dans la paroi abdominale d'un patient en position latérale gauche, caractérisé en ce que la poche triangulaire est géométriquement agencée de sorte que son grand côté s'étende vers la gauche (le site d'implantation étant situé à gauche) lorsque le dispositif est placé dans un plan vertical avec son petit côté vertical et sa pompe manuelle en partie basse et lorsqu'il est observé de face, côté paroi externe.

10. Dispositif de distribution selon l'une des revendications précédentes, caractérisé en ce que:
- le corps présente une épaisseur plus faible que les dimensions de sa section d'ouverture sur la face active dudit corps,
- ledit corps (10) est pourvu autour de la dépression d'une bordure périphérique sur laquelle est fixée de façon étanche le pourtour de la membrane de refoulement (12),

- ladite membrane de refoulement est sensiblement plane au repos.

11. Dispositif de distribution selon l'une des revendications 1, 2, 3, 4, 5, 6, 7, 8, 9 ou 10, caractérisé en ce que la dépression (11) du corps de pompe présente une forme oblongue symétrique par rapport au plan axial (C), dont les sections par des plans de coupe parallèles à la face active possèdent une surface décroissante au fur et à mesure que ces plans de coupe se rapprochent du fond de la dépression.

12. Dispositif de distribution selon l'une des revendications 1, 2, 3, 4, 5, 6, 7, 8, 9, 10 ou 11, caractérisé en ce que la face active du corps de pompe (10) et la membrane de refoulement (12) portée par celle-ci sont inclinées par rapport à la face opposée du corps, de sorte que ledit corps présente une épaisseur décroissante en direction du cathéter (20).

13. Dispositif de distribution selon l'une des revendications 10, 11 ou 12, caractérisé en ce que le corps de pompe (10) et sa dépression (11) possèdent une forme oblongue symétrique par rapport à un plan axial.

14. Dispositif de distribution selon l'une des revendications 10, 11, 12 ou 13, caractérisé en ce que:
- le corps de pompe (10) est moulé d'un seul tenant en matière synthétique rigide biocompatible, notamment polycarbonate ou silicone renforcé,
- la membrane de refoulement (12) est découpée à la forme de la bordure périphérique de la dépression (11) dans une matière synthétique élastique biocompatible, notamment silicone.

15. Dispositif de distribution selon la revendication 14, caractérisé en ce que la membrane de refoulement (12) est réalisée en une matière synthétique élastique du type "auto-étanche" en vue de conférer à la pompe une fonction accessoire de site d'accès d'injection chronique.

16. Dispositif de distribution selon les revendications 6 et 14 prises ensemble, caractérisé en ce que l'élément du cadre (4a) fixé sur la paroi souple externe (1a) possède une extension (4c) autour du corps (10) de pompe.

17. Dispositif de distribution selon l'une des revendications 10, 11, 12, 13, 14, 15 ou 16, caractérisé en ce que:
- les moyens de communication entre la chambre volumétrique (13) et le cathéter (20) comprennent un canal (16) percé dans le corps (10) et débouchant, d'une part, dans la dépression (11) au voisinage du fond de celle-ci, d'autre part, sur son chant périphérique à l'extérieur de la poche (1), l'extrémité du cathéter (20) étant fixée de façon étanche dans ledit canal,
- la valve unidirectionnelle à seuil (18) est logée dans ledit canal (16) et est adaptée pour ouvrir celui-ci au-dessus d'un seuil Pc sensiblement compris entre 50 et 60 millibars,
- les moyens de communication entre le réservoir (1) et la chambre volumétrique (13) comprennent un canal (15) percé dans le corps (10) et débouchant, d'une part, dans la dépression (11) au voisinage du fond de celle-ci, d'autre part, sur son chant périphérique entre les parois souples délimitant le réservoir (1a, 1b),
- la soupape de sécurité (17) est logée dans ledit canal (15) et est adaptée pour obturer la communication au-dessus d'une pression Pr dans le réservoir sensiblement comprise entre 20 et 30 millibars.

18. Dispositif de distribution selon l'une des revendications 10, 11, 12, 13, 14, 15, 16 ou 17, dans lequel:
- la dépression (11) possède un volume sensiblement compris entre 0,4 et 0,6 cm$^3$ en vue de délimiter avec la membrane de refoulement au repos une chambre volumétrique (13) de capacité correspondant à une embole,
- les deux parois souples (1a, 1b) délimitent un réservoir de capacité sensiblement comprise entre 30 cm$^3$ et 50 cm$^3$.

19. Dispositif de distribution selon la revendication 4, caractérisé en ce que le septum d'injection (7) du site de remplissage est constitué par deux membranes élastiques (7a, 7b) portées par l'anneau (6) de façon à être séparées par un vide d'air.

20. Dispositif de distribution selon la revendication 19, dans lequel:
- la plaque de butée (5) du site de remplissage est en un matériau rigide biocompatible, notamment polycarbonate,
- chaque membrane (7a, 7b) comprend un bourrelet périphérique (7c) et est formée en un matériau élastique biocompatible, notamment silicone,
- l'anneau (6) comprend deux éléments annulaires (6a, 6b) formant l'âme des bourrelets périphériques (7c) de membrane et constitués en un matériau rigide biocompatible, notamment polytétrafluoroéthylène.

21. Dispositif de distribution selon l'une des revendications précédentes, caractérisé en ce qu'il est entièrement recouvert d'un mince revêtement souple (22) en une matière biocompatible, en particulier en silicone.

**Patentansprüche**

1. Handbetätigbare Vorrichtung für sequentielle Dosierung zur Implantierung in einem zugänglichen subkutanen Bereich zwecks sequentieller Dosierung von Dosen eines flüssigen Stoffes, der Art umfassend:
- ein Reservoir (1) mit einem mehreren Dosen entsprechenden Fassungsvermögen,
- einen Füllbereich (2) des Reservoirs, der eine Füllkammer (8), ein Septum (7) für die Injektion von flüssigem Stoff in die besagte Kammer und Mittel (9) für die Verbindung zwischen der Füllkammer und dem Reservoir umfaßt,
- und eine Handpumpe (3) mit einer volumetrischen Kammer (13), deren Fassungsvermögen einer Dosis entspricht, Verbindungsmitteln (15) zwischen dem Reservoir und der besagten Kammer zwecks Füllung dieser Kammer und Verbindungsmitteln (16) zwischen der besagten volumetrischen Kammer und einem externen Katheter (20),

wobei die besagte Dosiervorrichtung dadurch gekennzeichnet ist,

(a) daß das Reservoir durch einen flexiblen Beutel (1) gebildet wird, der sich aus zwei Wänden zusammensetzt, und zwar einer sogenannten externen Wand (1a) und einer sogenannten internen Wand (1b), die sich gegenüber einander erstrecken und miteinander auf dichte Weise entlang ihres peripheren Randes verbunden sind,

(b) die Handpumpe einen starren, aus einem Stück gefertigten Körper (10) umfaßt, der:

- der Dicke nach mit einer Vertiefung (11) konkaver Form ausgehöhlt ist, wobei die besagte Vertiefung in einer Oberfläche des besagten Körpers, der sogenannten aktiven Oberfläche, mündet und dadurch die volumetrische Kammer (13) bildet,

- zwischen den beiden Wänden (1a, 1b) des das Reservoir bildenden flexiblen Beutels eingeschaltet ist,

- an seiner aktiven Oberfläche eine Pumpmembran (12) trägt, die die volumetrische Kammer abschließt und an der Seite der externen Wand (1a) angeordnet ist,

- und mit seiner gegenüberliegenden Oberfläche an der internen Wand befestigt ist,

(c) daß die Verbindungsmittel zwischen der volumetrischen Kammer (13) und Katheter (20) mit einem Einweg-Grenzdruckventil (18) ausgestattet sind, das so beschaffen ist, daß es den von der Kammer zu dem Katheter führenden Verbindungskanal öffnet, wenn besagte Kammer einem Überdruck ausgesetzt ist, der einen festgesetzten Grenzwert Pc übersteigt,

(d) daß die Verbindungsmittel zwischen Reservoir (1) und volumetrischer Kammer (13) mit den folgenden Teilen ausgestattet sind:

- einem Rückschlagventil (21), das Rückfluß der Flüssigkeit aus der Kammer in Richtung des Reservoirs verhindert,

- einem Sicherheitsventil (17), das so beschaffen ist, daß es Fluß aus dem Reservoir in Richtung der volumetrischen Kammer gestattet und den Verbindungskanal sperrt, falls der Überdruck in dem Reservoir einen Wert Pr übersteigt, der geringer ist als der Grenzwert Pc oder diesem gleich ist.

2. Dosiervorrichtung nach Anspruch 1, dadurch gekennzeichnet,

- daß der Pumpenkörper (10) zwischen den beiden flexiblen Wänden (1a, 1b) in Nähe ihres peripheren Randes eingeschaltet ist,

- daß die flexible externe Wand (1a) in Übereinstimmung mit der Pumpmembran mit einem Fenster versehen und in dichter Weise mit Hilfe der Ränder des besagten Fensters an Körper (10) befestigt ist.

3. Dosiervorrichtung nach einem der Ansprüche 1 oder 2, dadurch gekennzeichnet, daß der flexible Beutel (1) am Rande seiner flexiblen Wände (1a, 1b) durch einen peripheren Rahmen (4) versteift ist, der an dem Umfang der besagten flexiblen Wände angebracht ist.

4. Dosiervorrichtung nach einem der Ansprüche 1, 2 oder 3, dadurch gekennzeichnet,

- daß der Füllbereich (2) in der Nähe des peripheren Randes der flexiblen Wände (1a, 1b) angeordnet ist, und zwar in einem Bereich des flexiblen Beutels gegenüber jenem, in dem sich die Handpumpe (3) befindet,

- daß sich der Füllbereich aus einer starren Abstützplatte (5), die an der flexiblen internen Wand (1b) angebracht ist, und einem mit der besagten Platte fest verbundenen und das einspritzende Septum (7) abstützenden Ring (6) zusammensetzt, wobei dieses mit einem Fenster der flexiblen internen Wand (1a) übereinstimmt, das auf dichte Weise rings um den besagten Ring angebracht ist.

5. Dosiervorrichtung nach einem der Ansprüche 1, 2, 3 oder 4, dadurch gekennzeichnet,

- daß der flexible Beutel (1), der das Reservoir bildet, eine allgemein dreieckige Form besitzt,

- daß sich die Handpumpe (3) in der Nähe eines der Eckpunkte befindet und der Füllbereich (2) in der Nähe des anderen Eckpunkts.

6. Dosiervorrichtung nach den Ansprüchen 3 und 5 gemeinsam, dadurch gekennzeichnet,

- daß jede flexible Wand (1a, 1b) aus flexiblem biokompatiblem Kunststoff vorgeformt ist, vor allem aus verstärktem Silikon, so daß sie im Ruhezustand den Schnitt eines Domes aufweist,

- daß der periphere Rahmen (4) zwei Elemente (4a, 4b) aus halbstarrem biokompatiblem Kunststoff, vor allem Polytetrafluoräthylen, umfaßt, die gegenüber einander an der externen Wand (1a) und an der internen Wand (1b) angebracht sind,

- daß der Rahmen (4) und die Ränder der flexiblen Wände (1a, 1b) im Bereich der Winkel abgerundet sind.

7. Dosiervorrichtung nach einem der Ansprüche 5 oder 6, dadurch gekennzeichnet,

- daß der flexible Beutel (1), der das Reservoir bildet die allgemeine Form eines annähernd rechtwinkligen Dreiecks aufweist, wobei eine Seite (v) des rechten Winkels kürzer ist als die andere,

- daß sich die Handpumpe (3) in dem spitzen Winkel befindet, der durch die kurze Seite (v) und die Hypotenuse gebildet wird, und zwar in Nähe des entsprechenden Eckpunkts,

- daß sich der Füllpunkt (2) in dem spitzen Winkel befindet, der durch die lange Seite (h) und die Hypotenuse gebildet wird, und zwar in Nähe des entsprechenden Eckpunkts,

- daß Nahtlöcher (23) in dem Rande des flexiblen Beutels vorgesehen sind, und zwar einerseits in Nähe des rechten Winkels und andererseits in Nähe des spitzen Winkels in dem Rande der Handpumpe (3).

8. Dosiervorrichtung nach Anspruch 7 zur Implantierung in der Bauchwand eines Patienten in rechter Seitenlage, dadurch gekennzeichnet, daß der dreieckige Beutel geometrisch so beschaffen ist, daß seine lange Seite (h) sich (bei rechts befindlichem Implantierungsort (2)) nach rechts erstreckt, wenn die Vorrichtung in einer senkrechten Ebene mit ihrer kurzen Seite (v) senkrecht und ihrer Handpumpe (3) am unteren Ende angeordnet und von vorn mit Blick auf Außenwand (1a) betrachtet wird.

9. Dosiervorrichtung nach Anspruch 7 zur Implantierung in der Bauchwand eines Patienten in der linken Seitenlage, dadurch gekennzeichnet, daß der rechteckige Beutel geometrisch so beschaffen ist, daß sich bei links befindlichem Implantierungsort seine lange Seite nach links erstreckt, wenn die Vorrichtung in senkrechter Ebene mit ihrer kurzen Seite senkrecht und ihrer Handpumpe am unteren Ende angeordnet und von vorn mit Blick auf die externe Wand betrachtet wird.

10. Dosiervorrichtung nach einem der vorstehenden Ansprüche, dadurch gekennzeichnet,
- daß die Dicke des Körpers geringer ist als die Maße seines Öffnungsschnitts an der aktiven Oberfläche des besagten Körpers,
- daß der besagte Körper (10) rings um die Vertiefung mit einem peripheren Rand versehen ist, an dem der Umfang der Pumpmembran (12) auf dichte Weise angebracht ist,
- daß die besagte Pumpmembran im wesentlichen eben ist.

11. Dosiervorrichtung nach einem der Ansprüche 1, 2, 3, 4, 5, 6, 7, 8, 9 oder 10, dadurch gekennzeichnet, daß die Vertiefung (11) des Pumpenkörpers eine im Verhältnis zu der Axialebene (C) längliche symmetrische Form aufweist, deren durch Schnittebenen parallel zu der aktiven Oberfläche gebildete Querschnitte ein abnehmendes Flächenmaß aufweisen, je näher diese Schnittebenen dem Boden der Vertiefung sind.

12. Dosiervorrichtung nach einem der Ansprüche 1, 2, 3, 4, 5, 6, 7, 8, 9, 10 oder 11, dadurch gekennzeichnet, daß die aktive Oberfläche des Pumpenkörpers (10) und die dadurch abgestützte Pumpmembran (12) im Verhältnis zu der gegenüberliegenden Oberfläche des Körpers geneigt sind, so daß die Dicke des besagten Körpers in der Richtung auf Katheter (20) zu abnimmt.

13. Dosiervorrichtung nach einem der Ansprüche 10, 11 oder 12, dadurch gekennzeichnet, daß der Pumpenkörper (10) und seine Vertiefung (11) im Verhältnis zu einer axialen Ebene eine längliche symmetrische Form aufweisen.

14. Dosiervorrichtung nach einem der Ansprüche 10, 11, 12 oder 13, dadurch gekennzeichnet,
- daß der Pumpenkörper (10) kontinuierlich aus starrem biokompatiblem Kunststoff, insbesondere Polykarbonat oder verstärktem Silikon, geformt ist,
- daß die Pumpmembran (12) aus einem elastischen biokompatiblen Kunststoff, insbesondere Silikon, der Form des peripheren Randes der Vertiefung (11) entsprechend zugeschnitten ist.

15. Dosiervorrichtung nach Anspruch 14, dadurch gekennzeichnet, daß die Pumpmembran (12) aus einem elastischen Kunststoff "selbstsiegelnder" Art gefertigt ist, um der Pumpe die zusätzliche Funktion eines Zugangspunktes für chronische Injektion zu verleihen.

16. Dosiervorrichtung nach den Ansprüchen 6 und 14 gemeinsam, dadurch gekennzeichnet, daß das Rahmenelement (4a), das an der flexiblen externen Wand (1a) befestigt ist, rings um den Körper (10) der Pumpe eine Verlängerung (4c) aufweist.

17. Dosiervorrichtung nach einem der Ansprüche 10, 11, 12, 13, 14, 15 oder 16, dadurch gekennzeichnet,
- daß die Verbindungsmittel zwischen der volumetrischen Kammer (13) und dem Katheter (20) einen in dem Körper (10) vorgesehenen Kanal (16) umfassen, der einerseits in der Vertiefung (11) im Bereiche von deren Boden mündet, und andererseits in dessen peripheren Bereich an der Außenseite des Beutels (1), wobei das Ende des Katheters (20) auf dichte Weise in dem besagten Kanal befestigt ist,
- daß das Einweg-Grenzventil (18) sich in dem besagten Kanal (16) befindet und so beschaffen ist, daß es diesen oberhalb einer im wesentlichen zwischen 50 und 60 Millibar liegenden Grenze Pc öffnet,
- daß die Verbindungsmittel zwischen dem Reservoir (1) und der volumetrischen Kammer (13) einen in dem Körper (10) vorgesehenen Kanal (15) umfassen, der einerseits in Nähe von deren Boden in Vertiefung (11) mündet, und andererseits in seinem peripheren Bereich zwischen den das Reservoir abgrenzenden flexiblen Wänden (1a, 1b),
- daß sich das Sicherheitsventil (17) in dem besagten Kanal (15) befindet und so beschaffen ist, daß es die Verbindung sperrt, wenn ein im wesentlichen zwischen 20 und 30 Millibar betragender Druck Pr in dem Reservoir überstiegen wird.

18. Dosiervorrichtung nach einem der Ansprüche 10, 11, 12, 13, 14, 15, 16 oder 17, in dem
- die Vertiefung (11) ein im wesentlichen zwischen 0,4 und 0,6 cm³ betragendes Volumen besitzt, um im Ruhezustand gemeinsam mit der Pumpmembran eine volumetrische Kammer (13) mit einem einem Embolus entsprechenden Fassungsvermögen abzugrenzen,
- die beiden flexiblen Wände (1a, 1b) ein Reservoir mit einem im wesentlichen zwischen 30 cm³ und 50 cm³ betragenden Fassungsvermögen abgrenzen.

19. Dosiervorrichtung nach Anspruch 4, dadurch gekennzeichnet, daß das Einspritzseptum (7) des Füllbereichs durch zwei elastische Membranen (7a, 7b) gebildet wird, die durch den Ring (6) abgestützt werden, so daß sie durch ein Vakuum getrennt sind.

20. Dosiervorrichtung nach Anspruch 19, in der
- die Abstützplatte (5) des Füllbereichs aus einem starren biokompatiblen Material, insbesondere Polykarbonat, besteht,
- jede Membran (7a, 7b) einen peripheren Wulst (7c) umfaßt und aus einem biokompatiblen elastischen Material, insbesondere Silikon, geformt ist,
- der Ring (6) zwei Ringelemente (6a, 6b) umfaßt, die den Kern der peripheren Wulst (7c) der Membran bilden und aus einem starren biokompatiblen Material, insbesondere Polytetrafluoräthylen, gebildet sind.

21. Dosiervorrichtung nach einem der vorstehenden Ansprüche, dadurch gekennzeichnet, daß sie vollkommen mit einer dünnen flexiblen Hülle (22) aus einem biokompatiblen Material, insbesondere Silikon, überzogen ist.

## Claims

1. Manually activated device for sequential distribution, intended for implantation in a subcutaneous region accessible with a view for sequential dispensing of liquid substance doses, of the type comprising:
- a reservoir (1) with a capacity corresponding to several doses,
- a charging point (2) of the reservoir comprising a charging chamber (8), a septum (7) for the injection of liquid substance into said chamber and means (9) for communication between the charging chamber and the reservoir,
- and a manual pump (3) comprising a volumetric chamber (13) with a capacity corresponding to one dose, means (15) for communication between the reservoir and said chamber with a view to charging the latter and means (16) for communication between said volumetric chamber and an external catheter (20),
said dispensing device being characterised in that:

(a) the reservoir consists in a flexible bag (1) composed of two walls, a so-called external wall (1a) and a so-called internal wall (1b), extending opposite one another and joined one to the other in sealed manner along their peripheral rim,
(b) the manual pump comprises a rigid body (10) made in one piece, which
- is hollowed in its thickness by a depression (11) of concave form opening out at one face of said body, the so-called active face, with a view to forming the volumetric chamber (13),
- is interposed between the two walls (1a, 1b) of the flexible bag forming the reservoir,
- carries a press-back membrane (12) on its active face, which closes the volumetric chamber and is situated at the side of external wall (1a),
- and is secured by its opposite face to the internal wall,
(c) the means of communication between volumetric chamber (13) and catheter (20) are provided with a unidirectional pressure limit valve (18) capable of opening the communication duct from the chamber towards the catheter when said chamber is subjected to an excess pressure higher than a preset limit Pc,
(d) the communication means between reservoir (1) and volumetric chamber (13) are provided
- with a non-return flap (21) preventing the reflux of liquid from the chamber towards the reservoir,
- with a safety valve (17) designed to enable flow from the reservoir towards the volumetric chamber and to block the communication duct if the excess pressure within the reservoir exceeds a value pr lower than or equal to the limit Pc.

2. Dispensing device according to claim 1, characterised in that:
- the body of pump (10) is interposed between the two flexible walls (1a, 1b) near their peripheral rim,
- the flexible external wall (1a) is provided in correspondence with the press-back membrane with a window and is secured in sealed manner to body (10) by the edges of said window.

3. Dispensing device according to one of claims 1 or 2, characterised in that flexible bag (1) is made rigid at the rim of its flexible walls (1a, 1b) by a peripheral frame (4) secured to the periphery of said flexible walls.

4. Dispensing device according to one of claims 1, 2 or 3, characterised in that:
- charging point (2) is located close to the peripheral rim of flexible walls (1a, 1b) in a zone of the flexible bag opposite to that where manual pump (3) is located,
- said charging point consists in a rigid abutment plate (5) secured to the flexible internal wall (1b) and in a ring (6) firmly attached to that plate and supporting injection septum (7) in such a manner that the latter is caused to correspond with a window in flexible external wall (1a) fixed in sealed manner about said ring.

5. Dispensing device according to one of claims 1, 2, 3 or 4, characterised in that:
- flexible bag (1) forming the reservoir is of in general triangular shape,
- manual pump (3) is located in the vicinity of one of the apices and charging point (2) in the vicinity of another apex.

6. Dispensing device according to claims 3 and 5 jointly, characterised in that:
- each flexible wall (1a, 1b) is premoulded of biocompatible flexible synthetic material, in particular reinforced silicone, so that in a state of rest its section is that of a dome,
- peripheral frame (4) comprises two elements (4a, 4b) of semi-rigid biocompatible synthetic material, in particular polytetrafluoroethylene, secured opposite one another to external wall (1a) and to internal wall (1b), respectively,
- frame (4) and the rims of flexible walls (1a, 1b) are rounded in the region of the angles.

7. Dispensing device according to one of claims 5 or 6, characterised in that:
- flexible bag (1) forming the reservoir has the general shape of an approximately right-angled triangle, one side (v) of the right angle being shorter than the other,
- manual pump (3) is situated in the acute angle delimited by short side (v) and the hypothenuse, close to the corresponding apex,
- charging point (2) is situated in the acute angle delimited by the long side (h) and the hypothenuse, close to the corresponding apex,
- suture holes (23) are provided in the rim of the flexible bag, close to the right angle on the one hand, and close to the acute angle at the rim of manual pump (3) on the other hand.

8. Dispensing device according to claim 7, intended for implantation within the abdominal wall of a patient in right-hand lateral position, characterised in that the triangular bag is geometrically so designed that its large side (h) extends towards the right (implantation point (2) being on the right) when the device is placed in a vertical plane with its small side (v) vertical and its manual pump (3) at the bottom and when viewed from the front, facing external wall (1a).

9. Dispensing device according to claim 7, intended for implantation in the abdominal wall of a patient in left-hand lateral position, characterised in that the triangular bag is geometrically so designed that its large side extends towards the left (the implantation point being located on the left) when the device is placed in a vertical plane with its small side vertical and its manual pump at the bottom and when viewed from the front, facing the external wall.

10. Dispensing device according to one of the preceding claims, characterised in that:
- the body has a thickness lower than the dimensions of its opening section on the active face of said body,
- said body (10) is provided about the depression with a peripheral rim to which is secured in sealed manner the periphery of press-back membrane (12),
- said press-back membrane is, in a state of rest, substantially plane.

11. Dispensing device according to one of claims 1, 2, 3, 4 ,5, 6, 7, 8, 9 or 10, characterised in that depression (11) of the pump body has a symmetrical oblong shape in respect of axial plane (C), the sections of which produced by planes of intersection parallel to the active face exhibit a diminishing surface as these planes of intersection approach the base of the depression.

12. Dispensing device according to one of claims 1, 2, 3, 4, 5, 6, 7, 8, 9, 10 or 11, characterised in that the active face of pump body (10) and press-back membrane (12) supported by the latter are inclined in relation to the opposite face of the body, so that said body exhibits a thickness diminishing in the direction of catheter (20).

13. Dispensing device according to one of claims 10, 11 or 12, characterised in that pump body (10) and its depression (11) exhibit, in relation to an axial plane, a symmetrical oblong shape.

14. Dispensing device according to one of claims 10, 11, 12 or 13, characterised in that:
- pump body (10) is formed continuously from biocompatible rigid synthetic material, in particular polycarbonate or reinforced silicone,
- press-back membrane (12) is cut to the shape of the peripheral rim of depression (11) from a biocompatible elastic synthetic material, in particular silicone.

15. Dispensing device according to claim 14, characterised in that press-back membrane (12) is made of an elastic synthetic material of "self-sealing" type with a view to imparting to the pump a subordinate function of access point for chronic injection.

16. Dispensing device according to claims 6 and 14 jointly, characterised in that frame element (4a) secured to flexible external wall (1a) has an extension (4c) about body (10) of the pump.

17. Dispensing device according to one of claims 10, 11, 12, 13, 14, 15 or 16, characterised in that:
- the communication means between volumetric chamber (13) and catheter (20) comprise a duct (16) passing through body (10) and opening out, on the one hand, in depression (11) close to the bottom of the latter, and, on the other hand, in its peripheral region outside bag (1), the end of catheter (20) being secured in sealed manner within said duct,
- unidirectional limit valve (18) is located within said duct (16) and so designed as to open the latter above a limit Pc substantially comprised between 50 and 60 millibars,
- the means of communication between reservoir (1) and volumetric chamber (13) comprise a duct (15) passing through body (10) and opening out, on the one hand, in depression (11) close to the bottom of the latter, and, on the other hand, in its peripheral region between the flexible walls delimiting the reservoir (1a, 1b),
- safety valve (17) is located within said duct (15) and so designed as to block communication above a pressure Pr in the reservoir substantially comprised between 20 and 30 millibars.

18. Dispensing device according to one of claims 10, 11, 12, 13, 14, 15, 16 or 17, in which:
- depression (11) has a volume substantially comprised between 0.4 and 0.6 cm$^3$ with a view to, in a state of rest, delimiting together with the press-back membrane a volumetric chamber (13) of a capacity corresponding to one embolus,
- the two flexible walls (1a, 1b) delimit a reservoir with a capacity substantially comprised between 30 cm$^3$ and 50 cm$^3$.

19. Dispensing device according to claim 4, characterised in that injection septum (7) of the charging point is constituted by two flexible membranes (7a, 7b) supported by ring (6) so as to be separated by a vacuum.

20. Dispensing device according to claim 19, in which:

- abutment plate (5) of the charging point is made of a rigid biocompatible material, in particular polycarbonate,

- each membrane (7a, 7b) comprises a peripheral bead (7c) and is formed of an elastic biocompatible material, in particular silicone,

- ring (6) comprises two annular elements (6a, 6b) forming the core of peripheral beads (7c) of the membrane and made of a rigid biocompatible material, in particular polytetrafluoroethylene.

21. Dispensing device according to one of the preceding claims, characterised in that it is entirely lined with a thin flexible lining (22) made of biocompatible material, in particular silicone.

Fig. 1

EP 0 202 696 B1

Fig. 2

Fig. 3

# Fig. 4

# Fig. 5

EP 0 202 696 B1

Fig. 6a

Fig. 6b

Fig. 6c

Fig. 7